Europäisches Patentamt

European Patent Office

Office européen des brevets

(11) Publication number : **0 007 735**
**B2**

(12) # NEW EUROPEAN PATENT SPECIFICATION

(45) Date of publication of the new patent specification :
01.10.86

(51) Int. Cl.⁴ : **C 07 C148/00, C 07 C149/10//**
**C23F11/16**

(21) Application number : **79301343.4**

(22) Date of filing : **10.07.79**

(54) **Process for making sulfurized olefins.**

(30) Priority : **25.07.78 US 928035**

(43) Date of publication of application :
**06.02.80 Bulletin 80/03**

(45) Publication of the grant of the patent :
**08.06.83 Bulletin 83/23**

(45) Mention of the opposition decision :
**01.10.86 Bulletin 86/40**

(84) Designated contracting states :
**BE DE FR GB IT NL**

(56) References cited :
FR-A- 2 211 455
US-A- 2 249 312
US-A- 3 471 404
US-A- 3 697 499
US-A- 3 703 504

(73) Proprietor : **MOBIL OIL CORPORATION**
**150 East 42nd Street**
**New York New York 10017 (US)**

(72) Inventor : **Horodysky, Andrew Gene**
**139 Weston Drive**
**Cherry Hill, New Jersey08003 Camden (US)**
Inventor : **Landis, Sherwood**
**458 Queen Street**
**Woodbury, New Jersey 08096 Gloucester (US)**

(74) Representative : **West, Alan Harry**
**Mobil Court 3 Clements Inn**
**London WC2A 2EB (GB)**

EP 0 007 735 B2

Jouve, 18, rue St-Denis, 75001 Paris, France

## Description

The invention relates to the preparation of organic sulfide compounds having improved copper strip corrosion activity.

Various proposals have been made for producing organic sulfides by treating olefins with sulfur chlorides and then reacting the resulting intermediate with compounds containing sulfur. For example, organic polysulfides may be prepared by the sulfochlorination of olefins containing 6 or more carbon atoms and further treatment with inorganic higher polysulfides according to U.S. Patent No. 2 708 199. In addition, U.S. Patent No. 3 471 404 discloses that sulfurization reactions of this nature may be carried out by reacting a sulfochlorinated isobutylene intermediate product with a mixture of an alkali metal sulfide and free sulfur in a molar ratio of at least 2 : 1 followed by a further prolonged treatment with aqueous sodium hydroxide, apparently for reducing high chlorine contents, to produce extreme pressure additives. U.S. Patent No. 3 068 218 indicates that sulfochlorinated products of improved color may be obtained by sulfochlorinating polymers of propylene, etc. containing 8 or more carbon atoms in an aqueous reaction mixture and then sulfurizing the intermediate with a solution of sodium sulfide in water and isopropanol, producing products with sulfur contents of the order of 10 to 34 % by weight. In U.S. Patent No. 2 249 312, the sulfochlorinated adduct of amylene or higher olefins is treated with sodium sulfide and/or other alkaline compounds to produce stable products of relatively low sulfur content and generally high chlorine contents.

In Example XX of that U.S. Patent, a di-isobutylene-sulfur monochloride reaction product is treated with an aqueous solution of sodium hydroxide and sodium monosulfide that has already been used to finish a previous similar batch, and the product of that treatment is further treated with a fresh sodium hydroxide/sodium monosulfide solution to obtain a final product containing 4.5 % chlorine. The high chlorine content is undoubtedly due to the use of depleted sodium monosulfide solution in the reaction.

In general, prior art organic sulfide compounds have one or more of undesirable characteristics such as high cost, low sulfur content, high chlorine content and corrosive attack on metals and alloys used in machinery. Product having a chlorine content above 2 % and also those produced from sodium polysulfide reactants are usually rather corrosive. Some of these disadvantages can be overcome and organic sulfide compounds having improved properties, especially as to high sulfur content and more desirable corrosion characteristics, are obtained by the economical process described in U.S. Patent No. 3 703 504. In this process, the aqueous alkali metal monosulfide reactant employed in the final reaction is derived from a spent effluent stream resulting from hydrocarbon purification operations.

The present invention provides a process for the preparation of an organic sulfide which comprises reacting a sulfur halide selected from sulfur chlorides and sulfur bromides with an olefin having from 2 to 12 carbon atoms and reacting the product thereof with an alkali metal sulfide to form a sulfurized olefin characterized in that the sulfur halide/olefin reaction product is reacted in solution with at least a stoichiometric amount of alkali metal sulfide and that the sulfurized olefine obtained is further reacted with a separate portion of an aqueous solution comprising alkali metal sulfide in a ratio of from 0.2 mole to 1.0 mole of sulfide per mole of sulfurized olefin.

This invention may be used in conjunction with any processes that involve the sulfohalogenation of olefinic materials. Thus, it can be utilized in any of the aforementioned and other prior art processes in which olefins are sulfohalogenated and subsequently subjected to a sulfurization-dehalogenation reaction in the production of organic sulfides.

A wide variety of olefinic substances may be charged to the initial or sulfochlorination reaction including olefins with terminal or internal double bonds and containing from 2 to 12 carbon atoms per molecule in either straight or branched chain compounds such as ethylene, propylene, 1-butene, cis and trans-2-butene, isobutylene, diisobutylene, triisobutylene, the pentenes, the hexenes, the octenes and 1-decene. In general $C_{3-6}$ olefins or mixtures thereof are preferred for preparing sulfurized products for use as extreme pressure additives as the combined sulfur content of the product decreases with increasing carbon content, and the miscibility of the product with oil is lower in the case of propylene and ethylene derivatives.

In some embodiments of the invention, isobutylene is particularly preferred as the predominant olefinic reactant but it may be employed, desirably in major proportion in mixtures containing one or more other olefins ; moreover, the charge may contain substantial proportions of saturated aliphatic hydrocarbons such as methane, ethane, propane, butanes and pentanes. Such alkanes are preferably present in minor proportion in most instances to avoid unnecessary dilution of the reaction since they neither react nor remain in the products but are expelled in the off-gases or by subsequent distillation. However, mixed charges can substantially improve the economics of the present process since such streams are of lower value than a stream of relatively pure isobutylene.

Volatile olefins are often readily available in liquid form, and it is usually desirable to charge olefinic liquids which are vaporized by the heat of reaction, as such evaporation provides a substantial cooling effect that permits the flow of water for cooling the reactor to be reduced considerably for greater economy. Also there are indications that the use of a volatile liquid olefin reactant has the unexpected and desirable effect of lowering the viscosity of the final product.

The other reactant in the first stage is preferably sulfur monochloride ($S_2Cl_2$) ; but other similar

compounds such as sulfur dichloride and $S_3Cl_2$ as well as the corresponding but more expensive sulfur bromides may be employed in an amount which will provide a quantity of sulfur corresponding to desirable reactant ratios for sulfur monochloride. The molar ratio of olefin to sulfur monohalide may range from 1 : 1 to 1.65 : 1. In the case of butylenes and sulfur monochloride, the optimum ratio appears to be between about 1.55 : 1 and 1.60 : 1.

Although anhydrous reaction conditions are generally regarded as providing better results, a small amount of water ranging up to about 1 % of the weight of the sulfur halide may be present in the initial reaction ; however, it is usually preferred to keep the water content below about 0.5 % on that basis.

The sulfohalogenation reaction is exothermic, evolving $1.163 \times 10^6$ to $1.512 \times 10^8$ J/kg (500-650 B.t.u./lb.) in the case of isobutylene, and cooling is necessary to prevent the temperature from exceeding about 71.1 °C (160 °F) with resultant darkening of the product and some decrease in the yield. The preferred range of reaction temperature is from 48.9 to 57.2 °C (120 to 135 °F) and a temperature of about 51.7 °C (125 °F) appears to be the optimum. Typical reaction times for the initial stage of the process range from 1 to 15 hours.

The reaction pressure is not critical, and may be illustrated by pressures ranging from 101.3 to 790.8 kPa (0 to 100 psig) depending upon the reaction temperature and the volatility of the olefinic material.

The initial reaction is preferably catalyzed with a lower aliphatic alcohol containing from 1 to 4 carbon atoms, such as methanol, ethanol, propanol or isopropanol. Of these, methanol and ethanol are usually preferred, especially the former, and the amount of the alcohol used may range from 0.2 to 10 % of the weight of the sulfur chlorid, but quantities of the order of 0.5 to 3 % are usually preferred. While the catalytic alcohol may be introduced into the reactor in the liquid state, it is often more desirable to introduce it as a vapor.

Hydrogen chloride or bromide is evolved in the reaction and this gas is vented from the reactor. It may be recovered as hydrochloric or hydrobromic acid in a water absorption system.

In the second stage of the process of the present invention, an alkali metal sulfide is reacted with the adduct from the first stage. A lower aliphatic alcohol is generally added as a mutual solvent for the sulfurization de-chlorination or de-bromination reaction. Methanol, ethanol, propanol, butanol and isobutanol as well as mixtures thereof may be employed for the purpose, and isopropanol is preferred. Although a quantity of alcohol in an amount to 10 % of weight of the sulfohalogenation adduct provides adequate solvent action in the reaction mixture, surprising effects are obtained with larger proportions of the alcohol in the reaction mixture in that more alcohol up to a quantity of about 50 % of the weight of the adduct not only provides an unexpected increase in the reaction rate but also a striking improvement in sharply reducing the content of undesired chlorine or bromine in the final product while increasing its sulfur content. Thus it is desirable to charge at least about 20 % alcohol and the range of about 25 to 40 % is preferred. While larger proportions of alcohol may provide some additional benefit, the difficulty of handing and recovering the extra alcohol also increases.

In sulfurizing and dechlorinating or debrominating the sulfochlorination or sulfurbromination addition product, the aqueous alkali metal monosulfide solution is present in at least a stoichiometric quantity, and preferably a slight excess, of available alkali metal in order to remove most of the combined chlorine or bromine from the adduct. Stated another way, the mole ratio of metal sulfide to adduct will range from about 1.0 to about 1.2. In practice, the adduct or intermediate product from the sulfochlorination reaction is pumped into the solution of sodium monosulfide in water and the alcohol in an amount usually ranging from 2.52 to 2.70 kg of adduct per kg of the sodium sulfide (anhydrous basis) providing a slight excess of available sodium.

In general, this treatment of the adduct may be carried out at temperatures between 65.6° to 121.1 °C (150 and 250 °F) and the range between 76.7° and 90.6 °C (170 and 195 °F) is usually preferred. The reaction pressure may be subatmospheric or elevated up to more than 446.07 kPa (50 psig). For simplicity, it is usually preferable to carry out the reaction at reflux temperature of typically 79.4° to 85 °C (175 to 185 °F) under atmospheric pressure in a vessel equipped with a reflux condenser. In a typical production scheme, as disclosed for example in U.S. Patent No. 3 697 499, the polysulfurized olefin is reacted with alkali metal hydroxide to yield a product having good copper corrosion characteristics. While this treatment yields an excellent product for some applications, it has been found that an 8 hour reaction time with alkali metal hydroxide is difficult to perform and does not always produce a product possessing optimal extreme pressure/antiwear activity.

We have found that by adding to the adduct another portion of alkali metal sulfide, ranging from 0.2 to 1.0 mole of sulfide to 1.0 mole of adduct, a product having excellent copper corrosion activity is obtained. This alkali metal sulfide can be dissolved in water for addition to the adduct as a 5-25 % solution. Or, the aqueous alkali metal monosulfide reactant can be derived from a spent effluent stream resulting from hydrocarbon purification operations such as those described in U.S. Patent No. 3 703 504. In this step, a lower alkanol, such as isopropanol may be used as described hereinabove. That is, it is used in the quantities specified in the description of the second stage reaction of the adduct with metal sulfide. In general, however, the treatment is milder since the bulk of the reaction has already taken place. The reaction is carried out at from 65.6° to 110 °C (150 °F to 230 °F). The range from 73.9° to 87.8 °C (165 °F to 190 °F) is usually preferred. The reaction may be carried out at subatmospheric pressure or up to 446.07 kPa (50 psig). Preferably it is carried out at atmospheric pressure.

3

# 0 007 735

Examples 1 to 5

Table 1 summarizes the Examples, including proportions of reactants, reaction conditions and the yield, analysis and copper strip corrosion activity of the products. In Example 1, the sulfurized olefin is not subjected to the additional treatment with sodium sulfide according to the present invention. This Example is included for comparison purposes only. In Examples 2 to 5, the sulfurized olefin, sodium sulfide and 2-propanol were combined and stirred at the temperatures and for the times shown. The sulfurized olefin used was made in accordance with U.S. Patent No. 3 703 504, Example 1, which employs isobutylene as the olefin.

## Table 1

### Treatment of sulfurized olefin

| Example | 1 | 2 | 3 | 4 | 5 |
|---|---|---|---|---|---|
| Sulfurized Olefin, g. | 300. | 300. | 300. | 300. | 300. |
| $Na_2S \cdot 9H_2O$, g. | | 296. | 148. | 74. | 37. |
| Concentration % $Na_2S$ in water | | 25% | 25% | 12.5% | 6.3% |
| 2-Propanol, g. | | 150. | 100. | 75. | 50. |
| Sulfurization Time, hrs. | | 3 | 3 | 2 3/4 | 3 |
| Sulfurization Temp., °C/°F | | 82.2/180 | 82.2/180 | 85.6/186 | 85.6/186 |
| 2-Propanol Distillation, hrs. | | 1/2 | 1/2 | 1/3 | 1/3 |
| Number of Washes (Distilled water- equal volume) | 3 | 3 | 3 | 3 | 3 |
| Product Drying Final Temperature, °C/°F | 87.8/190 | 87.5/190 | 93.3/200 | 92.2/198 | 91.1/196 |
| Color | Bright orange | Bright orange | Yellow- orange | Bright orange | Bright orange |
| Final Yield, g. | 283. | 235.5 | 239. | 254. | 255. |

## Table 1 (Cont'd.)

### Treatment of sulfurized olefin

| Analyses | Original sample | Examples | | | | |
|---|---|---|---|---|---|---|
| | | 1 | 2 | 3 | 4 . | 5 |
| % Sulfur | 45.1 | 43.7 | 37.5 | 39.6 | 40.2 | 41.6 |
| % Chlorine | 0.38 | 0.33 | 0.12 | 0.14 | 0.13 | 0.17 |
| Specific Gravity | 1.1422 | 1.1308 | 1.0715 | 1.0823 | 1.1058 | 1.1223 |
| Cu Strip Corrosion[1] | 2E | 2E | 1A | 1A | 2A | 3A |

[1] Determined by placing 3 wt. % of the test compound in a 200 second solvent paraffinic neutral mineral oil and testing for 6 hours at 98.9 °C (210 °F) in accordance with ASTM Test Procedure D-130-9.

## Examples 6 to 10

Examples 6 to 10 are included to demonstrate the improved products obtained according to the process of the present invention as compared to the product made according to the prior art and the products obtained by varying the amounts of sodium sulfide used and the time of treatment of the sulfurized olefin in a single sodium sulfide treatment step. The product of Example 6 was made substantially in accordance with the procedure of Example 1 of U.S. Patent No. 3 703 504. The product of Example 7 was made by using twice the time and twice the concentration of sodium sulfide used in Example 6. The product of Example 8 was made by using the same time as in Example 6 but with twice the concentration of sodium sulfide. The products of Examples 9 and 10, made according to the process of the present invention, are identical to Examples 2 and 3 above. Examples 9 and 10 clearly show an increase in the ability to inhibit copper strip corrosion.

(See Table 2 page 5)

4

Table 2

| Example | 6 | 7 | 8 | 9 | 10 |
|---|---|---|---|---|---|
| Sulfur Monochloride/ isobutylene adduct, g. | 150 | 150 | 150 | 300 | 300 |
| $Na_2S \cdot 9H_2O$, g. | 150 | 300 | 300 | 296 | 148 |
| Concentration, % $Na_2S$ in water | 25 | 25 | 25 | 25 | 25 |
| 2-Propanol, g. | 150 | 150 | 105 | 150 | 100 |
| Sulfurization Time, Hrs. | 3 | 6 | 3 | 3 | 3 |
| Sulfurization Temp., °C/°F | 85/185 | 85/185 | 85/185 | 82.2/180 | 82.2/180 |
| 2-Propanol Distillation, Hrs. | 1/2 | 1/2 | 1/2 | 1/2 | 1/2 |
| Number of Washes (Distilled water-equal volume) | 3 | 3 | 3 | 3 | 3 |
| Product Drying Final Temp., °C/°F | 87.8/190 | 87.8/190 | 87.8/190 | 87.8/190 | 93.3/200 |
| Color | Bright orange | Bright orange | Bright orange | Bright orange | Yellow orange |

Table 2 (Cont'd)

| Analyses | 6 | 7 | 8 | 9 | 10 |
|---|---|---|---|---|---|
| % Sulfur | 40 | 45 | 40 | 37.5 | 39.6 |
| % Chlorine | 0.3 | 0.4 | 0.4 | 0.12 | 0.14 |
| Cu Strip Corrosion[1] | 3B | 3A | 3B | 1A | 1A |

[1] Determined by placing 3 wt. % of the text compound in a 200" solvent paraffinic neutral mineral oil and testing for 6 hours at 98.9 °C (210 °F) in accordance with ASTM D-130-9, as per the application.

## Claims

1. A process for the preparation of an organic sulfide which comprises reacting a sulfur halide selected from sulfur chlorides and sulfur bromides with an olefin having from 2 to 12 carbon atoms and reacting the product thereof with an alkali metal sulfide to form a sulfurized olefin, characterized in that the sulfur halide/olefin reaction product is reacted in solution with at least a stoichiometric amount of alkali metal sulfide and that the sulfurized olefin obtained is further reacted with a separate portion of an aqueous solution comprising an alkali metal sulfide in a ratio of from 0.2 to 1.0 mole of sulfide per mole of sulfurized olefin.

2. The process of claim 1 wherein the alkali metal sulfide is sodium sulfide.

3. The process of claim 2 wherein the alkali metal sulfide is sodium monosulfide.

4. The process of any preceding claim wherein the olefin is isobutylene.

5. The process of any preceding claim wherein the reaction of the sulfurized olefin with the alkali metal sulfide is carried out in the presence of a lower aliphatic alcohol.

6. The process of claim 5 wherein the lower aliphatic alcohol is isopropanol.

7. The process of any preceding claim wherein the reaction of the sulfur halide with the olefin is run at a temperature not greater than 71.1 °C (160 °F).

8. The process of claim 7 wherein the temperature is from 48.9° to 57.2 °C (120 °F to 135 °F).

9. The process of any preceding claim wherein the reaction of the sulfurized olefin with the alkali metal sulfide is run at a temperature of from 65.6° to 110 °C (150° to 230 °F).

10. The process of any preceding claim wherein the olefine to sulfur halide ratio is from 1 : 1 to 1.65 : 1.

11. The process of any preceding claim wherein the alkali metal sulfide for the reaction with the sulfurized olefin is derived from the spent effluent from hydrocarbon purification operations.

## Patentansprüche

1. Verfahren zur Herstellung eines organischen Sulfids, bei dem ein Schwefelhalogenid, ausgewählt aus Schwefelchloriden und Schwefelbromiden, mit einem Olefin umgesetzt wird, das 2 bis 12 Kohlenstoffatome aufweist, und das Produkt dieser Umsetzung mit einem Alkalimetallsulfid unter Bildung eines geschwefelten Olefins umgesetzt wird, dadurch gekennzeichnet, daß das Schwefelhalogenid/Olefin-Reaktionsprodukt in Lösung mit zumindest der stöchiometrischen Menge des Alkalimetallsulfids umgesetzt wird und daß das erhaltene geschwefelte Olefin weiterhin mit einem getrennten Anteil einer wässrigen Lösung umgesetzt wird, die ein Alkalimetallsulfid in einem Verhältnis von von 0,2 bis 1,0 Mol Sulfid pro Mol des geschwefelten Olefins enthält.

2. Verfahren nach Anspruch 1, worin das Alkalimetallsulfid Natriumsulfid ist.

3. Verfahren nach Anspruch 2, worin das Alkalimetallsulfid Natriummonosulfid ist.

4. Verfahren nach einem der vorstehenden Ansprüche, worin das Olefin Isobutylen ist.

5. Verfahren nach einem der vorstehenden Ansprüche, worin die Umsetzung des geschwefelten Olefins mit dem Alkalimetallsulfid in Gegenwart eines niederen aliphatischen Alkohols durchgeführt wird.

6. Verfahren nach Anspruch 5, worin der niedere aliphatische Alkohol Isopropanol ist.

7. Verfahren nach einem der vorstehenden Ansprüche, worin die Umsetzung des Schwefelhalogenids mit dem Olefin bei einer Temperatur von nicht höher als 71,1 °C (160 °F) abläuft.

8. Verfahren nach Anspruch 7, bei dem die Temperatur von 48,9 bis 57,2 °C (120 bis 135 °F) beträgt.

9. Verfahren nach einem der vorstehenden Ansprüche, worin die Umsetzung des geschwefelten Olefins mit dem Alkalimetallsulfid bei einer Temperatur von von 65,6 bis 110 °C (150 bis 230 °F) abläuft.

10. Verfahren nach einem der vorstehenden Ansprüche, worin das Verhältnis von Olefin zu Schwefelhalogenid von 1 : 1 bis 1,65 : 1 beträgt.

11. Verfahren nach einem der vorstehenden Ansprüche, worin das Alkalimetallsulfid zur Umsetzung mit dem geschwefelten Olefin aus den verbrauchten Abwässern von Kohlenwasserstoff-Reinigungsverfahren stammt.

## Revendications

1. Un procédé amélioré pour la préparation d'un sulfure organique qui comprend la réaction d'un halogénure de soufre choisi parmi les chlorures de soufre et les bromures de soufre avec une oléfine ayant 2 à 12 atomes de carbone et la réaction du produit obtenu avec un sulfure de métal alcalin pour former une oléfine sulfurée, caractérisé en ce que l'on fait réagir le produit de la réaction halogénure de soufre/oléfine en solution avec au moins une quantité stœchiométrique de sulfure de métal alcalin et en ce que l'on fait encore réagir l'oléfine sulfurée obtenue avec une portion séparée d'une solution aqueuse comprenant un sulfure de métal alcalin dans un rapport de 0,2 à 1,0 mol de sulfure par mole de l'oléfine sulfurée.

2. Le procédé de la revendication 1, dans lequel le sulfure de métal alcalin est le sulfure de sodium.

3. Le procédé de la revendication 2, dans lequel le sulfure de métal alcalin est le monosulfure de sodium.

4. Le procédé de l'une quelconque des revendications précédentes, dans lequel l'oléfine est l'isobutylène.

5. Le procédé de l'une quelconque des revendications précédentes, dans lequel la réaction de l'oléfine sulfurée avec le sulfure de métal alcalin est effectuée en présence d'un alcool aliphatique inférieur.

6. Le procédé de la revendication 5, dans lequel l'alcool aliphatique inférieur est l'isopropanol.

7. Le procédé de l'une quelconque des revendications précédentes, dans lequel la réaction de l'halogénure de soufre avec l'oléfine est effectuée à une température ne dépassant 71 °C.

8. Le procédé de la revendication 7, dans lequel la température est entre 48,9 °C et 57,2 °C (120-135 °F).

9. Le procédé de l'une quelconque des revendications précédentes, dans lequel la réaction de l'oléfine sulfurée avec le sulfure de métal alcalin est effectuée à une température de 65,6 à 110 °C (150-230 °F).

.10. Le procédé de l'une quelconque des revendications précédentes, dans lequel le rapport de l'oléfine à l'halogénure de soufre est de 1/1 à 1,65/1.

11. Le procédé de l'une quelconque des revendications précédentes, dans lequel le sulfure de métal alcalin pour la réaction avec l'oléfine sulfurée dérive de l'effluent résiduaire d'opérations de purification d'hydrocarbures.